# EUROPÄISCHE PATENTANMELDUNG

(11) **EP 1 548 125 A1**
(43) Veröffentlichungstag der Anmeldung: **29.06.2005**
(21) Anmeldenummer: 03029692.5
(22) Anmeldetag: 23.12.2003
(51) Int. Cl.: C12Q 1/68

(54) **Verfahren und Testkit zur Genotypisierung des Prionprotein-Gens bei Schafen und Ziegen**

(71) Anmelder: G.A.G Bioscience GmbH, 28359 Bremen (DE)
(72) Erfinder: Mosner, Jörn, Dr., 28357 Bremen (DE); Helmer, Georg, Dr., 44892 Bochum (DE)
(74) Vertreter: Baumbach, Friedrich, Dr.

(57) **Zusammenfassung**

Die Erfindung betrifft ein Verfahren und ein Testkit zur Genotypisierung des für das Prionprotein-kodierenden Gens bei Schafen (Wiederkäuern). Durch die Analyse spezieller Punktmutationen wird die individuelle Anfälligkeit, in Gegenwart des infektiösen Scrapie-assoziierten Prionproteins (PrP^{Sc}) an Scrapie zu erkranken, nachgewiesen. Für Scrapie anfällige Tiere können so von der Zucht ausgeschlossen werden, was letztendlich zu Scrapie-resistenten Rassen führt. Anwendungsgebiete sind die Medizin und die Landwirtschaft und hier besonders die Tierzucht.

SEQUENZPROTOKOLL

<110> G.A.G. Bioscience GmbH

<120> Verfahren und Testkit zur Genotypisierung des
      Prionprotein-Gens bei Schafen und Ziegen

<130> 03029692.5

<140> 03029692.5
<141> 2003-12-23

<160> 7

<170> PatentIn Ver. 2.1

<210> 1
<211> 407
<212> DNA
<213> Ovis sp.

<220>
<223> Teilsequenz des PrP-Gens; Wildtyp

<400> 1
gccaaaaacc aacatgaagc atgtggcagg agctgctgca gctggagcag tggtaggggg 60
ccttggtggc tacatgctgg gaagtgccat gagcaggcct cttatacatt ttggcaatga 120
ctatgaggac cgttactatc gtgaaaacat gtaccgttac cccaaccaag tgtactacag 180
accagtggat cagtatagta accagaacaa ctttgtgcat gactgtgtca acatcacagt 240
caagcaacac acagtcacca ccaccaccaa gggggagaac ttcaccgaaa ctgacatcaa 300
gataatggag cgagtggtgg agcaaatgtg catcacccag taccagagag aatcccaggc 360
ttattaccaa aggggggcaa gtgtgatcct cttttcttcc cctcctg 407

<210> 2
<211> 407
<212> DNA
<213> Ovis sp.

<220>
<223> Teilsequenz des Prionprotein-Gens; Punktmutation
      an Position 87 der Teilsequenz (c wird ersetzt
      durch t); führt zum Austausch der Aminosäure 136
      des Prionproteins (A wird ersetzt durch V)

<400> 2
gccaaaaacc aacatgaagc atgtggcagg agctgctgca gctggagcag tggtaggggg 60
ccttggtggc tacatgctgg gaagtgtcat gagcaggcct cttatacatt ttggcaatga 120

ctatgaggac cgttactatc gtgaaaacat gtaccgttac cccaaccaag tgtactacag 180
accagtggat cagtatagta accagaacaa ctttgtgcat gactgtgtca acatcacagt 240
caagcaacac acagtcacca ccaccaccaa gggggagaac ttcaccgaaa ctgacatcaa 300
gataatggag cgagtggtgg agcaaatgtg catcacccag taccagagag aatcccaggc 360
ttattaccaa aggggggcaa gtgtgatcct cttttcttcc cctcctg 407

<210> 3
<211> 407
<212> DNA
<213> Ovis sp.

<220>
<223> Teilsequenz des Prionprotein-Gens; Punktmutation
      an Position 141 der Teilsequenz (g wird ersetzt
      durch a); führt zum Austausch der Aminosäure 154
      des Prionproteins (R wird ersetzt durch H)

<400> 3
gccaaaaacc aacatgaagc atgtggcagg agctgctgca gctggagcag tggtaggggg 60
ccttggtggc tacatgctgg gaagtgccat gagcaggcct cttatacatt ttggcaatga 120
ctatgaggac cgttactatc atgaaaacat gtaccgttac cccaaccaag tgtactacag 180
accagtggat cagtatagta accagaacaa ctttgtgcat gactgtgtca acatcacagt 240
caagcaacac acagtcacca ccaccaccaa gggggagaac ttcaccgaaa ctgacatcaa 300
gataatggag cgagtggtgg agcaaatgtg catcacccag taccagagag aatcccaggc 360
ttattaccaa aggggggcaa gtgtgatcct cttttcttcc cctcctg 407

<210> 4
<211> 407
<212> DNA
<213> Ovis sp.

<220>

<400> 4
gccaaaaacc aacatgaagc atgtggcagg agctgctgca gctggagcag tggtaggggg 60
ccttggtggc tacatgctgg gaagtgccat gagcaggcct cttatacatt ttggcaatga 120
ctatgaggac cgttactatc gtgaaaacat gtaccgttac cccaaccaag tgtactacag 180
accagtggat cggtatagta accagaacaa ctttgtgcat gactgtgtca acatcacagt 240
caagcaacac acagtcacca ccaccaccaa gggggagaac ttcaccgaaa ctgacatcaa 300
gataatggag cgagtggtgg agcaaatgtg catcacccag taccagagag aatcccaggc 360
ttattaccaa aggggggcaa gtgtgatcct cttttcttcc cctcctg 407

<210> 5
<211> 407
<212> DNA

<213> Ovis sp.

<220>
<223> Teilsequenz des Prionprotein-Gens; Punktmutation
      an Position 193 der Teilsequenz (g wird ersetzt
      durch t); führt zum Austausch der Aminosäure 171
      des Prionproteins (Q wird ersetzt durch H)

<400> 5
gccaaaaacc aacatgaagc atgtggcagg agctgctgca gctggagcag tggtaggggg 60
ccttggtggc tacatgctgg gaagtgccat gagcaggcct cttatacatt ttggcaatga 120
ctatgaggac cgttactatc gtgaaaacat gtaccgttac cccaaccaag tgtactacag 180
accagtggat cattatagta accagaacaa ctttgtgcat gactgtgtca acatcacagt 240
caagcaacac acagtcacca ccaccaccaa gggggagaac ttcaccgaaa ctgacatcaa 300
gataatggag cgagtggtgg agcaaatgtg catcacccag taccagagag aatcccaggc 360
ttattaccaa aggggggcaa gtgtgatcct cttttcttcc cctcctg 407

<210> 6
<211> 20
<212> DNA
<213> Ovis sp.

<220>
<223> Oligonukleotid; Primer Sip up (5'-3')

<400> 6
gccaaaaacc aacatgaagc 20

<210> 7
<211> 20
<212> DNA
<213> Ovis sp.

<220>
<223> Oligonukleotid; Primer Sip dn (5'-3')

<400> 7
caggagggga agaaaagagg 20

## Beschreibung

Die Erfindung betrifft ein Verfahren und ein Testkit zur Genotypisierung des für das Prionprotein-kodierenden Gens bei Schafen und Ziegen. Durch die Analyse spezieller Punktmutationen wird die individuelle Anfälligkeit, in Gegenwart des infektiösen Scrapie-assoziierten Prionproteins (PrP^{Sc}) an Scrapie zu erkranken, nachgewiesen. Für Scrapie anfällige Tiere können so von der Zucht ausgeschlossen werden, was letztendlich zu Scrapie-resistenten Rassen führt. Anwendungsgebiete sind die Medizin und die Landwirtschaft und hier besonders die Tierzucht.

Scrapie, auch als "Traberkrankheit des Schafs" bezeichnet, ist eine ansteckende und tödlich verlaufende Hinkrankheit, die Schafe und Ziegen befällt. Die Krankheit ist seit über 250 Jahren in Europa endemisch und wird von Tier zu Tier als auch vom Mutterschaf auf das Lamm übertragen. Als neurodegenerative Prionenerkrankung gehört Scrapie neben der bovinen spongiformen Enzephalopathie (BSE) des Rindes und der Creutzfeld-Jakob-Krankheit des Menschen zur Gruppe der transmissiblen spongiformen Enzephalopathien (TSE). In Großbritannien fiel sehr früh das Auftreten der Erkrankung in bestimmten Rassen und Linien auf, und man vermutete schon 1755, dass die Krankheitsentwicklung von genetischen Faktoren abhängt. Im Zeitraum von 1960 - 1980 wurden in Großbritannien Herden verschiedener Schafrassen etabliert, in denen Schafe nach experimenteller Infektion auf Scrapie-Resistenz oder -Empfänglichkeit selektiert werden konnten. Die Ergebnisse dieser Studien wiesen übereinstimmend darauf hin, dass Resistenz und Empfänglichkeit gegenüber Scrapie hauptsächlich von einem einzelnen Gen kontrolliert wird und Scrapie eine Infektionskrankheit ist, deren Inzidenz von der Anwesenheit des Erregers und der genetischen Empfänglichkeit des Tieres abhängt.

Nachdem Prusiner 1982 postuliert hatte, dass Scrapie und andere TSEs durch Veränderung körpereigener Prionpreteine (PrP) verursacht werden, wurden bei der Suche nach dem verantwortlichen Gen schließlich Assoziationen zwischen Mutationen im PrP-Gen von Schafen und Scrapie-Resistenz oder-Empfänglichkeit sowohl nach natürlicher als auch nach experimenteller Infektion nachgewiesen, wobei schon hier Unterschiede zwischen Schafrassen auffielen. Das PrP-Gen des Schafes besteht aus etwa 20.000 DNA-Bausteinen (Nukleotiden) und beinhaltet drei Exons.

Der das Protein codierende Bereich liegt im dritten Exon und bestimmt mit 768 Nukleotiden die Abfolge der 256 Aminosäuren im PrP-Protein, wobei immer drei Nukleotide als sogenannte Codons vorgeben, welche Aminosäure an dieser Position im Protein eingebaut wird.

Bis heute wurden eine Reihe von Variationen auf der Ebene der Nukleotide (Basenaustausche) im ovinen PrP-Gen und als Folge davon auch in der Aminosäuresequenz (Aminosäurenpolymorphismen) des Prionproteins beschrieben. Weiterhin existieren auch Variationen anderer Regionen des PrP-Gens, deren möglicher Einfluss auf TSE-Empfänglichkeit derzeit ebenfalls untersucht wird.

Bisher gelten die Aminosäurenpolymorphismen an den Positionen 136, 154 und 171 als am wichtigsten für Scrapie-Resistenz und -Empfänglichkeit. Für den Austausch von Alanin (A) und Valin (V) an der Position 136 bzw. von Arginin (R) mit Histidin (H) an der Position 154 ist jeweils das zweite Nukleotid im entsprechenden Codon zuständig, während für den Austausch von Glutamin (Q) an der Position 171 mit Arginin (R) das zweite Nukleotid und für den Austausch mit Histidin (H) zusätzlich das dritte Nukleotid des Codons verantwortlich ist.

| **Position der Aminosäure** | **Nukleinsäure -Sequenz** | **Aminosäure** | **Zustand** |
|---|---|---|---|
| 136 | gcc | Alanin (A) | Wildtyp |
| | gtc | Valin (V) | Mutation |
| | | | |
| 154 | cgt | Arginin (R) | Wildtyp |
| | cat | Histidin (H) | Mutation |
| | | | |
| 171 | cag | Glutamin (Q) | Wildtyp |
| | cgg | Arginin (R) | Mutation |
| | cat | Histidin (H) | Mutation |

Nicht alle theoretisch möglichen Kombinationen (Allele) der drei Codons konnten bisher gefunden werden. Neben dem Wildtyp A₁₃₆R₁₅₄Q₁₇₁ (einfache Schreibweise: ARQ) wurden folgende Allele beschrieben: ARR, ARH, AHQ, VRQ sowie selten AHR, VRR und noch seltener ARK und TRQ.

Der PrP-Genotyp ergibt sich aus den jeweiligen Allelen auf den beiden homologen Chromosomen, so dass bei Nachkommen unterschiedliche Genotypen auftreten können.

Dabei sind bestimmte Allele wie beispielsweise ARQ und je nach Schafrasse auch ARR sehr häufig, andere wie AHQ und VRQ kommen in bestimmten Rassen häufiger und andere wie AHR und VRR sehr selten vor, wohingegen solche wie beispielsweise VHQ noch bei keinem Schaf gefunden wurden.

Auch bei der Ziege sind Variationen im PrP-Gen beschrieben worden. Dabei zeigten sich Zusammenhänge zwischen bestimmten genetischen Varianten und der Inkubationszeit nach Infektion mit dem Scrapie-Erreger, allerdings bisher nur unter experimentellen Bedingungen.

Durch den weltweiten Vergleich der Genotypen (Codons 136, 154 und 171) von Scrapie (TSE)-positiven und Scrapie (TSE)-negativen Schafen in natürlich infizierten Herden konnte festgestellt werden, dass Schafe mit dem Genotyp ARR/ARR (homozygot) mit einer wissenschaftlich umstrittenen Ausnahme zeitlebens nie an Scrapie (TSE) erkrankten, während andere Genotypen in unterschiedlicher Häufigkeit Scrapie-Empfänglichkeit zeigten. Vor allem in Großbritannien wurden daraufhin Systeme zur Klassifizierung von Genotypen bezüglich ihrer Scrapie-Empfänglichkeit entwickelt. Die Einteilung der Genotypen in die Risikoklassen R1 (sehr niedriges Risiko) bis R5 (höchstes Risiko) richtet sich dabei nach dem Vorkommen entsprechender Allele in einzelnen Rasse, wobei in allen Rassen das ARR-Allel im Rahmen natürlicher Infektionen Resistenz vermittelt, der Grad der Empfänglichkeit der anderen Allele allerdings variiert.

Insgesamt werden die größten Unterschiede im Grad der Empfänglichkeit der verschienen Geotypen zwischen den Rassen mit wenigen verschiedenen Genotypen (sogenannte einfache Rassen wie Suffolk aus Großbritannien) und denen mit sehr vielen verschiedenen Genotypen (sogenannte komplexe Rassen wie Texel) beobachtet. So findet man in Rassen, in denen das VRQ-Allel vorkommt, bei Scrapie-positiven Tieren immer mindestens ein VRQ-Allel, während in Rassen, in denen das VRQ-Allel und auch das AHQ-Allel nicht oder nur sehr selten vorkommen, die von Scrapie betroffenen Schafe meistens den Genotyp ARQ/ARQ oder seltener ARR/ARQ haben. An diesem Beispiel wird auch deutlich, das ein schützender Effekt ("Dominanz") des ARR-Allels, der in bestimmten Rassen dazu führt, dass Tiere mit wenigstens einem ARR-Allel nicht an Scrapie erkranken (z.B. Cheviot, American Suffolk), in anderen Rassen nicht bestätigt werden kann (Texel, Scottish Suffolk, Ile de France).

Innerhalb der Rassen mit Variationen an der Position 136 (AN) kann man zwischen Gruppen unterschieden, bei denen Schafe, die an der Position 136 homozygot für Alanin (A) sind, nie an Scrapie erkranken (z.B. Cheviot, Ile de France) und anderen, bei denen auch solche Tiere gelegentlich an Scrapie erkranken (z.B. Texel, Lacaune, Romanov). Ähnliches wird für das AHQ-Allel beschrieben, welches in Rassen mit höherer VRQ-Frequenz Resistenz vermittelt (Cheviot, Texel, Islandschaf), während es in anderen Rassen (Romanov, Suffolk, Finn Dorset) sowohl bei Scrapie-positiven Schafen als auch bei gesunden Tieren gefunden wird.

Die Gründe für die dargestellten Rasseunterschiede im Grad der Empfänglichkeit bestimmter PrP-Genotypen sind noch nicht geklärt. Diskutiert werden als Ursache die unterschiedlichen Allel-Frequenzen bzw. die Anwesenheit oder das Fehlen bestimmter Allele in einer Population und eine mögliche Anpassung von verschiedenen Erregerstämmen an verschiedene Genotypen.

Im Experiment lässt sich eine lebenslange Resistenz von oral mit dem Scrapieoder auch mit dem BSE-Erreger infizierten Schafen mit dem Genotyp ARR/ARR bestätigen, während die intrazerebrale Infektion auch bei diesen Tieren zu einer Erkrankung geführt haben soll. Schafe mit anderen Genotypen erkranken dagegen nach oraler Infektion innerhalb von unterschiedlich langen Inkubationszeiten und zeigen unterschiedliche Konzentrationen und Verteilungen des Erregers im Gewebe. Dabei wurden ähnliche Beobachtungen gemacht wie bei den natürlichen Infektionen, d.h. Schafe mit dem Genotyp VRQNRQ erkranken nach einer kürzeren Inkubationszeit als solche mit dem Genotyp ARR/VRQ.

Auch bei den deutschen Schafrassen gibt es große Unterschiede im Vorkommen und der Frequenz der PrP-Allele. Das bezüglich einer möglichen Zucht auf Scrapie (TSE)-Resistenz besonders interessante ARR-Allel kommt in vielen Fleischrassen wie Dt. Schwarzköpfiges Fleischschaf, Dt. Weißköpfiges Fleischschaf und Suffolk und auch bei einigen Landrassen wie dem Rhönschaf bei über 50 % der Tiere vor. Bei anderen Rassen ist das ARR-Allel mit einer mittleren Häufigkeit vertreten (z.B. über 30 % bei Texel und 25 % bei der Grauen Gehörnten Heidschnucke). Ungünstiger sind die züchterischen Voraussetzungen bei Rassen wie dem Merinolandschaf, dem Weißen Bergschaf (Frequenz des ARR-Allels 11 % bzw. 3,5 %, Genotypisierungsergebnisse aus Bayern, 2002) und dem Ostfriesischen Milchschaf (ca. 9 %) aufgrund des niedrigen Anteils an Tieren mit mindestens einem ARR-Allel.

Innerhalb von Rassen können z.T. deutliche Unterschiede bei den Allelfrequenzen auftreten, so beispielsweise zwischen verschiedenen Regionen und auch zwischen einzelnen Herden.

Bezüglich der Vielfalt der PrP-Genotypen ist auch in Deutschland das Texel-Schaf mit 17 beschriebenen Genotypen unübertroffen, gefolgt von Suffolk und Weißem Bergschaf mit jeweils 11 verschiedenen Genotypen, während man beim Dt. Schwarzköpfigen Fleischschaf bisher nur 5 verschiedene Genotypen gefunden hat. Als in Deutschland gezüchtete Schafrassen mit einem wesentlichen VRQ-Anteil sind neben Texel zahlenmäßig unbedeutendere und zum Teil importierte Rassen wie Kamerunschaf und Gotlandschaf zu nennen. Als Besonderheit findet man in Deutschland mit dem Merinolandschaf, der Grauen Gehörten Heidschnucke und dem Ostfriesischen Milchschaf auch Rassen, die einen höheren AHQ-Anteil (bis 30 % beim Ostfriesischen Milchschaf) in Verbindung mit einem unbedeutenden VRQ-Anteil aufweisen.

Auch länderübergreifend werden Unterschiede bei den Genotypfrequenzen gefunden, wie beispielsweise bei Suffolks, bei denen in Irland zur zwei Allele (ARR und ARQ) gehäuft vorkommen, während in Deutschland abhängig von der Region bis zu 6 verschiedene Allele beschrieben wurden.

Die genetische Situation bezüglich der PrP-Allel- und Genotypfrequenzen bei deutschen Schafrassen macht deutlich, dass bei der Einschätzung der Scrapie (TSE)-Empfänglichkeit einzelner Genotypen (außer bei ARR/ARR) die Rasse oder Rassengrundlage einer von Scrapie (TSE) betroffenen Herde berücksichtigt werden müßte. Bei derzeitigem Vorgehen im Seuchenfall können neben allen ARR/ARR-Schafen auch Mutterschafe und Schlachttiere von der Tötung ausgenommen werden, die mindestens ein ARR-Allel sowie kein VRQ-Allel tragen. Dabei werden mit hoher Wahrscheinlichkeit Rassebesonderheiten wie vorhandene oder nicht vorhandene "Dominanz" des ARR-Allels über empfänglichere Allele oder gesteigerte Empfänglichkeit des AHQ-Allels bei gleichzeitigem Fehlen des VRQ-Allels in der Population nicht berücksichtigt. Deshalb ist es unbedingt notwendig, im Bezug auf Scrapie (TSE)-Empfänglichkeit eine differenzierte Betrachtung der einzelnen Rassen unter Einbeziehung der TSE-Situation nicht nur in Deutschland sondern europaweit durchzuführen, da die derzeitige Risikobewertung der PrP-Genotypen zumindest für bestimmte Rassen zu pauschal ist und einer Überarbeitung bedarf.

Zur Feststellung des PrP-Genotyps eines Tieres wird eine Blut- oder Gewebeprobe benötigt. Nach der Isolierung der DNA sind verschiedene Methoden der Genotypisierung möglich, die sich hinsichtlich des Zeit- und Kostenaufwandes und der Quantität und Qualität der gewonnenen Informationen unterscheiden.
Durch Sequenzierung kann ein gesamter Genbereich bezüglich der Abfolge seiner Nukleotide untersucht werden, während beispielsweise durch Restriktionsspaltung, Massenspektrometrie oder Minisequenzierung nur einzelne Codons typisiert werden. Die Bestimmung des Haplotyps, also der Kombination einzelner Allele, ist mit Hilfe der Sequenzierung sehr aufwendig. Darüber hinaus sind die bisherigen Methoden zur Genotypisierung sehr zeitund kostenintensiv und mit einem großen apparativen Aufwand verbunden und es wird Personal benötigt, das die Geräte (Sequenzierautomat, Massenspektrometer) sicher bedienen kann.

Die Aufgabe der vorliegenden Erfindung war es, ein Verfahren sowie ein Testkit zu entwickeln, mit dem eine schnelle und kostengünstige Genotypisierung des für das Prionprotein-kodierenden Gens bei Schafen möglich ist.

Die Erfindung wird durch die Ansprüche realisiert.

Gegenstand der Erfindung ist ein Verfahren und ein Testkit zur Genotypisierung des Prionprotein-Gens bei Schafen. Das Verfahren ist gekennzeichnet durch folgende Verfahrensschritte:
- Isolierung von genomischer DNA aus einem geeigneten Gewebe des zu untersuchenden Tieres;
- Anreicherung eines Genfragments des Prionprotein-Gens mit Hilfe der PCR;
- Hybridisierung das angereicherten Genfragments mit markierten universellen Sonden (Signalsonden) und immobilisierten allelspezifischen Sonden (Nachweissonden);
- Bestimmung des variablen Nukleotids (Punktmutation) durch eine mismatch-sensitive Ligation der Nachweis- und Signalsonden;
- Binden eines Konjugats an die Markierung der verbliebenen legierten Signalsonden;
- Nachweis des gebundenen Konjugats durch Hinzugabe eines Substrats und Messung der Farbreaktion;
- Bestimmung des Genotyps durch Auswertung der Farbreaktion.

Der Testkit zur Genotypisierung des Prionprotein-Gens bei Schafen besteht mindestens aus:
- einem Pimerpaar für die PCR-Reaktion zur Anreicherung eines Genfragments des Prionprotein-Gens, wobei als Primer bevorzugt Oligonukleotide mit folgenden Sequenzen eingesetzt werden: Primer Sip up (5'-3') GCCAAAAACCAACATGAAGC Primer Sip dn (5'-3') CAGGAGGGGAAGAAAAGAGG
- markierte universelle Sonden (Signalsonden);
- an ein geeignetes Trägermaterial gebundene allelspezifische Sonden (Nachweissonden);
- eine DNA-Ligase;
- ein Konjugat, das an die Markierung der Signalsonden binden kann;
- ein Substrat, das durch das Konjugat zu einem Farbumschlag führt.

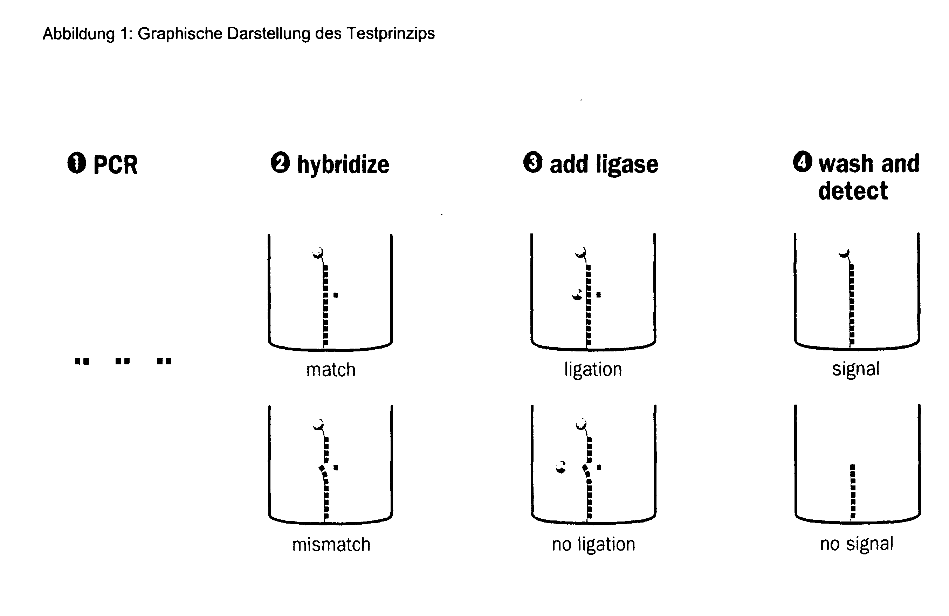

Die Signalsonden sind komplementär zu Teilsequenzen des Prionprotein-Gens, die sich unmittelbar vor oder nach den zu untersuchenden Nukleinsäuren (Punktmutation) befinden und sind an dem Ende markiert, welches sich nicht unmittelbar vor oder nach der zu untersuchenden Nukleinsäuren befindet (Abb.1).

Die Nachweissonden sind komplementär zu Teilsequenzen des Prionprotein-Gens, wobei die Nachweissonde mit dem zu untersuchenden Nukleotid endet.

Die Nachweissonden sind mit dem Ende an das Trägermaterial gebunden, das nicht mit dem zu untersuchenden Nukleotid endet.

Das erfindungsgemäße Verfahren wird im folgenden näher erläutert. Im ersten Schritt wird das relevante Genfragment mit einer PCR aus genomischer DNA angereichert. Genomische DNA wird aus der biologischen Probe mittels etablierter Verfahren isoliert. Für die Genfragment-Amplifikation in der PCR werden Primer und Wasser im Kit mitgeliefert.

Im zweiten Schritt bildet das PCR-Fragment mit markierten universellen Sonden (=Signalsonden) und immobilisierten allelspezifischen Sonden (=Nachweissonden) in den Vertiefungen der Nachweisplatte einen Hybridisierungskomplex.
Ein Gemisch aus Signalsonden und Hybridisierungspuffer wird dem PCR-Fragment zugesetzt. Die resultierende PCR-Fragmentlösung wird in eine Probenspur der Nachweisplatte verteilt, die mit normal- und mutationssequenzspezifischen Nachweissonden beschichtet ist.
Es bildet sich ein Hybridisierungskomplex aus einem Strang des PCR-Fragments, der Nachweissonde und der Signalsonde. Die Sonden hybridisieren direkt nebeneinander. Dabei liegt das variable Nukleotid des PCR-Fragments an der Übergangsstelle.

Im dritten Schritt wird das variable Nukleotid des PCR-Fragments durch eine Mismatch-sensitive Ligation der Nachweis- und Signalsonden mit einer DNA-Ligase bestimmt. Eine DNA-Ligase wird dem Hybridisierungskomplex zugegeben. So können Nachweis- und Signalsonden enzymatisch zu einem Molekül verbunden werden. Dies erfolgt nur, wenn das variable Nukleotid des PCR-Fragments zur Nachweissonde komplementär ist.
Je nachdem welche Genotypen in den PCR-Fragmenten vorliegen, kann entweder ausschliesslich die normalsequenzspezifische Nachweissonde (=homozygot normaler Genotyp), sowohl die normal- als auch die mutationssequenzspezifische Nachweissonde (heterozygoter Genotyp), oder ausschliesslich die mutationssequenzspezifische Nachweissonde mit der Signalsonde verbunden werden.

Im vierten Schritt wird ein Konjugat an die ligierten Signalsonden gebunden. Das Konjugat katalysiert den Farbumschlag eines Substrats. Durch Waschen werden PCR-Fragmente und nicht-ligierte Signalsonden entfernt. Danach wird ein Konjugat an die Markierung der verbliebenen ligierten Signalsonden gebunden.
Durch erneutes Waschen wird überschüssiges Konjugat entfernt und spezifisch gebundenes Konjugat durch die Zugabe eines colorimetrischen Substrats nachgewiesen. Dadurch entsteht in den Vertiefungen der Nachweisplatte eine Farbreaktion, in denen korrespondierende PCR-Fragmente und Nachweissonden vorhanden waren. Der Farbumschlag tritt bereits nach Minuten ein und kann einfach visuell ausgewertet werden. Insgesamt werden nach erfolgter PCR etwa 1 Stunde und 15 Minuten für die Genotyp-Bestimmung benötigt.

Ausführungsbeispiel:

| **Bestandteile des Testkits** | |
|---|---|
| 1 | 96-well-MTP mit allelspezifischen Sonden, *gebrauchsfertig* |
| 1 ml | Wasser, PCR-grade |
| für 10 PCRs | PCR-Primer, *lyophilisierf* |
| für je 8 wells | Normale and mutierte Kontroll-DNAs, *lyophilisiert* |
| für 1 Platte | Signalsonden, *lyophilisiert* |
| 0,34 ml | Hybridisierungspuffer |
| für 1 Platte | Ligase-Enzym, *lyophilisiert* |
| 11 ml | Ligationspuffer |
| 31 ml | Waschlösung, *gebrauchsfertig* |
| 11 ml | Konjugatpuffer |
| für 1 Platte | Avidin-Peroxidase-Konjugat, *gebrauchsfertig* |
| 1,1 ml | Substratkomponente A |
| 11 ml | Substratkomponente B |
| 1,5 ml | Stopplösung, *gebrauchsfertig* |

### Nähere Beschreibung der Testkit-Bestandteile

96-well-MTP mit allelspezifischen
Sonden (Nachweissonden)

PCR-Primer | Primer 1 (5'-3') GCCAAAAACCAACATGAAGC
 | Primer 2 (5'-3') CAGGAGGGGAAGAAAAGAGG
Ligase-Enzym | T4 DNA-Ligase

### Durchführung

### 1. Amplifikation des relevanten Genfragments in der PCR

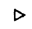 genomische DNA mit etabliertem Verfahren isolieren und auf etwa 20 ng/µl TE oder Wasser einstellen.
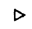 Lyophilisierte PCR-Primer aus dem Kit mit 390 µl Wasser rekonstituieren.
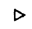 PCR-Mastermix auf Eis ansetzen. Einzelne PCRs bestehen aus 50 µl-Ansätzen:

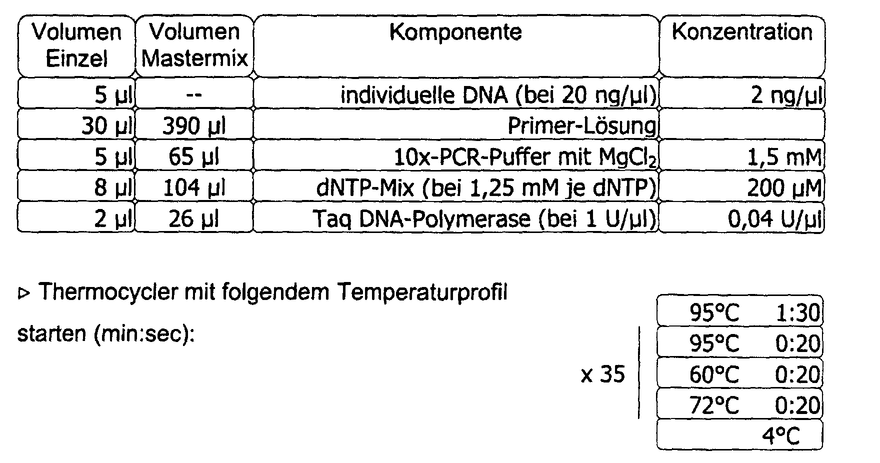

### 2. Kit-Vorbereitung und Hybridisierung

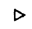 Kontroll-DNAs mit 50 µl Wasser rekonstituieren.
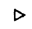 Signalsonde mit 25 µl Wasser rekonstituieren, in Hybridisierungspuffer überführen (→ Hybridisierungslösung).
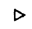 Ligase-Enzym mit 100 µl Wasser rekonstituieren, in Ligationspuffer überführen (→ Ligaselösung).
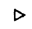 Konjugat in Konjugatpuffer überführen (→ Konjugatlösung).
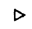 Substratkomponente A in B überführen (→ Substratlösung).
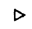 10 µl der Hybridisierungslösung direkt den PCR-Gefässen und den Kontroll-DNAs zusetzen.

Durch mehrmaliges auf- und abpipettieren mischen (→ PCR-Fragmentlösungen).
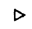 5 µl/well jeder individuellen PCR-Fragmentlösung in eine Probenspur der Nachweisplatte pipettieren.
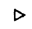 15 Minuten hybridisieren.

### 3. Ligation der Nachweis- und Signalsonden

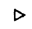 100 µl/well Ligaselösung in die Probenspur zugeben. 15 Minuten ligieren.

### 4. Colorimetrischer Nachweis ligierter Signalsonden

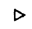 Platte entleeren.
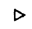 100 µl/well Waschlösung einfüllen und entleeren.
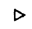 100 µl/well Konjugatlösung einfüllen. 30 Minuten inkubieren.
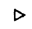 Platte entleeren.
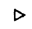 100 µl/well Waschlösung einfüllen und entleeren (1. Waschschritt).
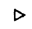 100 µl/well Waschlösung einfüllen und entleeren (2. Waschschritt).
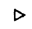 100 µl/well Substratlösung einfüllen und 10-30 Minuten Farbe entwickeln.
10 µl/well Stopplösung zugeben (optional) und die Genotypen entweder visuell,
in einem ELISA-Reader
bei 405 nm, oder mit einer Digitalkamera auswerten und dokumentieren.

### Allgemeine Hinweise zum Test und zur Testdurchführung

Zur Bereitstellung amplifizierbarer genomischer DNA können etablierte Verfahren oder kommerziell erhältliche Kits verwendet werden.
Abhängig von der Ausbeute genomischer DNA kann es notwendig sein, das Rekonstitutionsvolumen der lyophilisierten PCR-Primer zu reduzieren. Bei suboptimaler DNA-Qualität kann es vorteilhaft sein, die PCR-Gefäße direkt vom Eis in den hochgeheizten Block des Thermocyclers zu stellen. Das empfohlene Temperatur-Profil ist für dünnwandige PCR-Gefässe mit einem Nennvolumen von 200 µl und ölfreie Durchführung mit Deckelheizung optimiert. Bei Verwendung dickwandiger Gefässe oder eines Öl-Overlay sollten die Reaktionszeiten verlängert werden. Die verwendete Taq DNA-Polymerase und der Thermocycler können die Ausbeuten der PCR beeinflussen.
Die strenge Separierung der Reagenzien und Pipetten des sauberen "Prä-PCR"-Laborbereiches vom allgemeinen "Post-PCR-"Laborbereich ist essentiell, um eine Kontamination mit PCR-Fragmenten zu vermeiden. Kontroll-PCRs ohne genomische-DNA mit anschliessender Analyse durch Gelelektrophorese oder wird empfohlen.

Eine eingefärbte Taq DNA-Polymerase (RedTaq, Sigma Kat.Nr. D4309) erleichtert nicht nur die visuelle Kontrolle der Pipettierschritte, sondern ist nach unser Erfahrung auch am reproduzierbarsten einzusetzen.

Der Assay wird bei Raumtemperatur auf der Laborwerkbank ohne Schüttler oder Inkubatoren durchgeführt.
Repetier- und Multichannel-Pipetten sowie Pipettierautomaten sind in allen Schritten einsetzbar.
Auf die vollständige Rekonstitution der lyophilisierten Kit-Komponenten ist zu achten. Insbesondere bei der Rekonstitution des Ligase-Enzyms sollte die gesamte Wandfläche des Gefässes mehrmals benetzt werden.
Kontroll-DNAs sind synthetische DNA-Fragmente, die wie PCR-Fragmente eingesetzt werden.
Das Substrat ABTS (2,2 Azino-bis(3-Ethylbenzthiazoline-6-Sulfonic Acid)) sollte zu Beginn der Farbreaktion nur leicht grünlich sein. Die Farbentwicklung ist linear, so dass ein Stoppen nicht unbedingt notwendig ist. Gestoppte und abgedeckte Nachweisplatten können bei Raumtemperatur bis zu 2 Tage aufbewahrt und abgelesen werden.
Für hohen Probendurchsatz sind stabilisierte Flüssigreagenzien in grösseren Gebinden erhältlich, bei denen die Rekonstitution entfällt.
Das erfindungsgemäße Verfahren sowie das Testkit ermöglicht eine schnelle und einfache Genotypisierung des für das Prionprotein-kodierenden Gens bei Schafen. Das Verfahren kann selbst von unerfahrenem Laborpersonal sicher durchgeführt werden und liefert innerhalb weniger Stunden das Ergebnis.
Vor allem die enorme Kostenersparnis im Vergleich zu herkommlichen Genotypisierungsmethoden macht dieses Verfahren für den Einsatz in der Tierzucht interessant. Durch die erfindungsgemäße Genotypisierung, die gegenüber bisher verwendeten Verfahren schnell, mit deutlich geringeren Kosten und ohne großen apparativen und personellen Aufwand durchzuführen ist, wird erstmals eine effektive Selektion von Scrapie-resistenten und Scrapiesensitiven Tieren ermöglicht. Diese Selektion ist die Voraussetzung für eine Zucht von Scrapie-resistenten Schafrassen, die wiederum eine erfolgreiche Bekämpfung dieser Erkrankung ermöglicht.

Legende zu den Abbildungen:

Abbildung 1: Graphische Darstellung des Testprinzips

**Schritt** 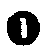

Im ersten Schritt wird das relevante Genfragment mit einer PCR aus genomischer DNA angereichert.

**Schritt** 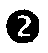

Im zweiten Schritt bildet das PCR-Fragment mit markierten universellen Sonden (=Signalsonden) und immobilisierten allelspezifischen Sonden (=Nachweissonden) in den Vertiefungen der Nachweisplatte einen Hybridisierungskomplex.

**Schritt** 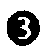

Im dritten Schritt wird das variable Nukleotid des PCR-Fragments durch eine Mismatch-sensitive Ligation der Nachweis- und Signalsonden mit dem Enzym T4 DNA-Ligase bestimmt.

**Schritt** 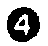

Im vierten Schritt wird ein Streptavidin-Peroxidase-Konjugat an die ligierten Signalsonden gebunden. Das Konjugat katalysiert den grünen Farbumschlag eines Substrats.

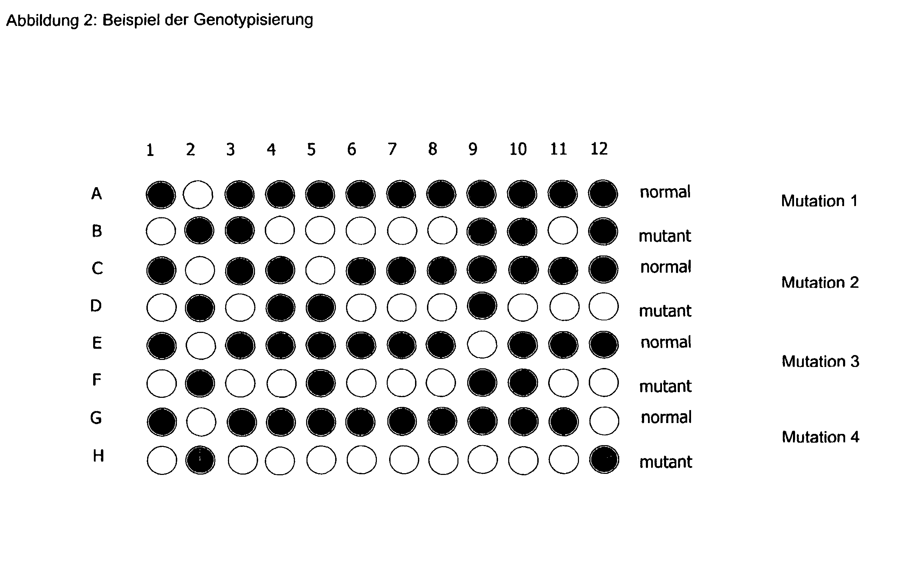

### Abbildung 2: Beispiel der Genotypisierung

In den Probenspuren 1 und 2 wurde jeweils die normale und mutierte Kontroll-DNA analysiert.

Ein homozygot normaler Genotyp wurde für Mut. 1 in den Proben 4-8 und 11, für Mut. 2 in den Proben 3, 6-8 und 10-12, für Mut. 3 in den Proben 3-4, 6-8 und 11-12, für Mut. 4 in den Proben 3-11 nachgewiesen.

Ein heterozygoter Genotyp wurde für Mut. 1 in den Proben 3, 9-10 und 12, für Mut. 2 in den Proben 4 und 9, für Mut. 3 in den Proben 5 und 10 nachgewiesen. Ein homozygot mutierter Genotyp wurde für Mut. 2 in der Probe 5, für Mut. 3 in der Probe 9, für Mut. 4 in der Probe 12 nachgewiesen.

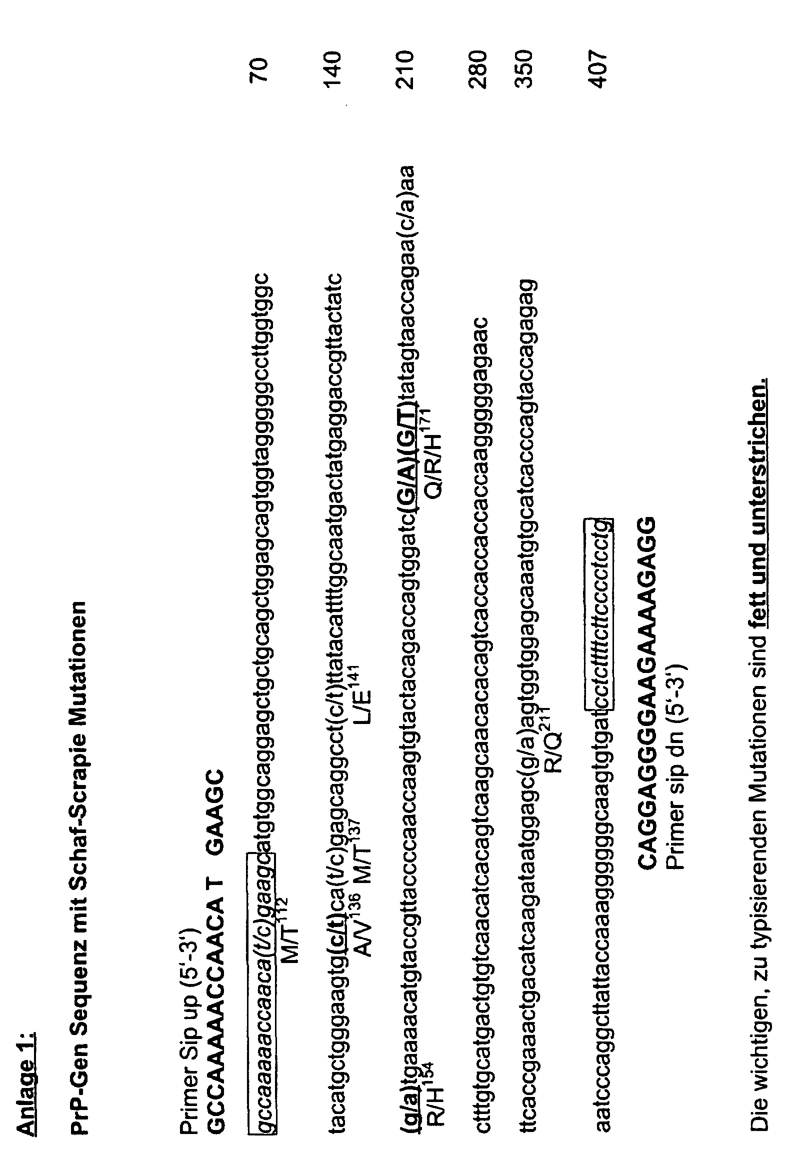

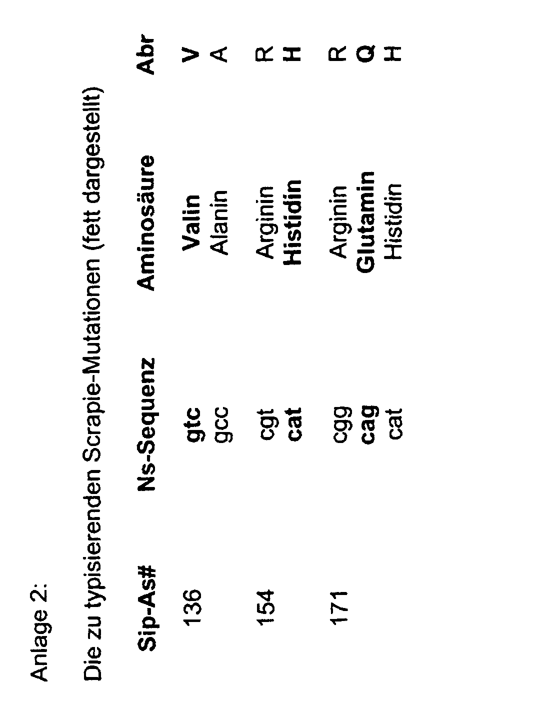

## Patentansprüche

1. Verfahren zur Genotypisierung des Prionprotein-Gens bei Schafen **gekennzeichnet durch** folgende Verfahrensschritte:
- Isolierung von genomischer DNA aus einem geeigneten Gewebe des zu untersuchenden Tieres;
- Anreicherung eines Genfragments des Prionprotein-Gens mit Hilfe der PCR;
- Hybridisierung das angereicherten Genfragments mit markierten universellen Sonden (Signalsonden) und immobilisierten allelspezifischen Sonden (Nachweissonden);
- Bestimmung des variablen Nukleotids (Punktmutation) **durch** eine mismatch-sensitive Ligation der Nachweis- und Signalsonden;
- Binden eines Konjugats an die Markierung der verbliebenen legierten Signalsonden;
- Nachweis des gebundenen Konjugats **durch** Hinzugabe eines Substrats und Messung der Farbreaktion;
- Bestimmung des Genotyps **durch** Auswertung der Farbreaktion.

2. Testkit zur Genotypisierung des Prionprotein-Gens bei Schafen mindestens bestehend aus:
- einem Pimerpaar für die PCR-Reaktion zur Anreicherung eines Genfragments des Prionprotein-Gens;
- markierte universelle Sonden (Signalsonden);
- an ein geeignetes Trägermaterial gebundene allelspezifische Sonden (Nachweissonden);
- eine DNA-Ligase;
- ein Konjugat, das an die Markierung der Signalsonden binden kann;
- ein Substrat, das durch das Konjugat zu einem Farbumschlag führt.

3. Testkit gemäß Anspruch 2 **dadurch gekennzeichnet, dass** als Primer Oligunukleotide mit folgenden Sequenzen eingesetzt werden:

Primer Sip up (5'-3') GCCAAAAACCAACATGAAGC

Primer Sip dn (5'-3') CAGGAGGGAAGAAAAGAGG

4. Testkit gemäß Anspruch 2 bis 3 **dadurch gekennzeichnet,**
**dass** die Signalsonden komplementär zu Teilsequenzen des Prionprotein-Gens sind, die sich unmittelbar vor oder nach den zu untersuchenden Nukleinsäuren (Punktmutation) befinden.

5. Testkit gemäß Anspruch 2 bis 4 **dadurch gekennzeichnet,**
**dass** die Signalsonden an dem Ende markiert sind, das sich nicht unmittelbar vor oder nach der zu untersuchenden Nukleinsäuren befindet.

6. Testkit gemäß Anspruch 2 bis 5 **dadurch gekennzeichnet,**
**dass** die Nachweissonden komplementär zu Teilsequenzen des Prionprotein-Gens sind, wobei die Nachweissonde mit dem zu untersuchenden Nukleotid endet.

7. Testkit gemäß Anspruch 2 bis 6 **dadurch gekennzeichnet,**
**dass** die Nachweissonden mit dem Ende an das Trägermaterial gebunden sind, das nicht mit dem zu untersuchenden Nukleotid endet.

8. Testkit gemäß Anspruch 2 bis 7 **dadurch gekennzeichnet,**
**dass** die Signalsonden mit Biotin markiert sind und als Konjugat ein Streptavidin-Konjugat Anwendung findet.
